# EUROPEAN PATENT SPECIFICATION

(11) **EP 3 140 655 B1**
(45) Date of publication and mention of the grant of the patent: **12.12.2018**
(21) Application number: 15751091.8
(22) Date of filing: 07.05.2015
(51) Int. Cl.: G01N 33/574

(54) **METHOD FOR DETECTING CARCINOGENESIS IN THE UTERINE CERVIX**
VERFAHREN ZUR DETEKTION VON CARCINOGENESE IN DER GERBÄRMUTTERHALS
PROCÉDÉ DE DÉTECTION DE LA CARCINOGENESE DANS LE COL DE L'UTERUS

(30) Priority: 07.05.2014 IT RM20140225
(43) Date of publication of application: 15.03.2017
(73) Proprietor: Maccallini, Vincenzo, 67051 Avezzano (AQ) (IT); Di Benedetto, Giuseppe, 81100 Caserta (CE) (IT)
(72) Inventor: Maccallini, Vincenzo, 67051 Avezzano (AQ) (IT); Di Benedetto, Giuseppe, 81100 Caserta (CE) (IT)
(74) Representative: Comoglio, Elena
(86) International application number: PCT/IT2015/000126
(87) International publication number: WO 2015/170360

(56) References cited:
- WO-A1-2010/129821
- WO-A1-2011/084598
- WANG P ET AL: "[Study on the carcinogenic mechanism of human papillomavirus typel6 E7 protein in cervical carcinoma]", ZHONGHUA SHIYAN HE LINCHUANG BINGDUXUE ZAZHI - CHINESE JOURNAL OF EXPERIMENTAL AND CLINICAL VIROLOGY, ZHONGHUA YIXUEHUI BINGHU XUEHUI, BEIJING, CN, vol. 14, no. 2, 1 June 2000 (2000-06-01), pages 117-120, XP008174761, ISSN: 1003-9279
- WANG P ET AL: "[Relationship between human papillomavirus E7 protein and Rb gene product in fresh tissues of squamous cervical carcinoma]", CHUNG-HUA FU CH'AN K'O TSA CHIH - CHINESE JOURNAL OF OBSTETRICSAND GYNECOLOGY, CHINESE MEDICAL JOURNALS PUBL. HOUSE, CN, vol. 32, no. 12, 1 December 1997 (1997-12-01), pages 722-724, XP008174751, ISSN: 0529-567X

## Description

### Field of the invention

The present invention concerns a method for detecting carcinogenesis in the uterine cervix. More specifically, the invention concerns some procedures for diagnosing cancer of the uterine cervix which integrate or surrogate the field of action of cytology with that of molecular biology, and provide a system for diagnosis, prognosis and management of HPV-induced cervical lesions which exploits very sensitive and specific analytical methods, such as Western Blot and Sandwich ELISA, in order to detect those cases where the transformation towards a neoplastic lesion has become irreversible.

### Background of the invention

The association between human papillomavirus (HPV) infection and cancer has stimulated an ongoing interest in researchers. HPV infects basal cells of the squamous epithelium and induces the formation of benign lesions and/or, in some cases, invasive malignant tumors. The HPV virion consists of a protein coat (capsid) surrounding a circular double-stranded DNA organized in coding and non-coding regions. In the coding region eight early open read ing frames (ORF) (E1-E8) and two late ORFs (LI, L2) have been identified. The early ORFs encode proteins involved in viral DNA replication during the proliferation, the regulation of viral gene expression (E2), the virus assembly (E4), the virus immortalization and transformation (E6 and E7 - only in high-risk HPVs). Late ORFs are activated only after cell differentiation and encode viral capsid proteins (LI and L2). The promoters, enhancers and other regulatory elements are located in the non-coding Upstream Regulatory Region (URR).

All HPV types develop and reproduce solely in keratinocytes (or keratin-producing epithelial cells), whose differentiation is critical to the viral development. Upon passing through a lesion of the epithelial layers, HPV reaches and infects the keratinocyte stem cells located in the basal layer of the epithelium. Here the virus begins its replication using the cell's replication machinery: it reproduces its own genome several times while retaining a low viral load (up to 50-300 copies). When the basal cells proliferate and move towards the outer layers of the epithelium, at the same time also the viruses move, and continue to reproduce without further amplifying their genome, so as to escape detection by the immune system. When the host keratinocyte reaches S-phase of its differentiation (keratinization), the HPV replicates its genome up to about 1000 copies. In fact, at this stage the virus releases E6 and E7 proteins, which stimulate the cell passage to the S-phase (Kadaja M, et al., Papillomavirus DNA replication - from initiation to genomic instability. Virology. 2009 Feb. 20; 384(2):360-8). Finally, when the keratinocyte reaches the superficial epithelium and dies, the viral genome, repackaged into a capsid, comes out of the cell.

In industrialized countries cervical cancer is a carefully controlled disease, thanks to the dissemination of Pap test and particularly to organized screening programs. Actually, this is one of the few oncological diseases that can be prevented through early detection and treatment of pre-invasive lesions (Cervical Intraepithelial Neoplasia, CIN). Through cytological examination of a small amount of cells taken from the cervix it is possible to determine the presence of anomalies in the squamous epithelium (SIL, Squamous Intraepithelial Lesions), that are classified as ASC-US (Atypical Squamous Cells of Undetermined Significance), ASC-H (Atypical Squamous Cells - cannot exclude HSIL), LSIL (Low-grade Squamous Intraepithelial Lesion), HSIL (High-grade Squamous Intraepithelial Lesion) and, finally, "carcinoma", when carcinomatous cells are already present. It has been estimated that Pap test screening every three years in the age group of 35-65 years can reduce the risk of invasive cancer by 90% (IARC Working Group on the Evaluation of Cancer Preventive Strategies. IARC Handbooks of Cancer Prevention Vol. 10. Cervix Cancer Screening Lyon: IARC press, 2005).

In the last decades HPV has been recognized as a necessary but not sufficient cause of cervical cancer (IARC Working Group **2005,** loc. cit.). Only persistent infection by high-risk types of HPV can cause cancer (Schiffman M, et al. Lancet. 2007; 370:890-907). The International Agency for Research on Cancer (I.A.R.C.) has recently confirmed its statements on carcinogenicity of some types of HPV (Strait KA, et al., on behalf of the WHO International Agency for Research on Cancer Monograph Working Group. Lancet Oncol. 2009; 10:321-322), and identified 12 high-risk types plus one suspect type, while 12 other types have an uncertain relationship with the tumor. This important advance in knowledge of the natural history of the disease has rapidly led to the introduction of two new preventive tools: the vaccine to prevent the infection itself and the test for detecting DNA of high oncogenic risk HPV strains (hr-HPV DNA testing) to identify women who have, or risk to develop, pre-invasive or invasive lesions. The hr-HPV DNA test has proven to be more sensitive than cytology in cervical screening (Arbyn J. et al. Vaccine. 2006; 24 Suppl 3: S3-78-89; Cuzick J. et al. lnt J Cancer. 2006; 119:1095-1101).

However, neoplastic transformation is a rare complication of infection by high-risk HPV which, in the majority of cases, is a transient event. Moreover, it has been shown that low-grade lesions (CIN 1, that correspond to LSIL of Pap test), which are also related to HPV infection, cannot be regarded as true pre-invasive lesions because in the vast majority of cases they spontaneously regress (Schiffman M. et al. Lancet. 2007; 370:890-907). For this reason, therefore, the hr-HPV DNA test has low specificity (Cuzick J. et al. **2006,** *loc. cit.).*

Randomized controlled trials have shown the efficacy of hr-HPV DNA testing in reducing both mortality and incidence of cervical cancer (Sankaranarayanan R. et al. N Engl J Med. 2009; 360:1385-94; Bulkmans NW. et al. Lancet. 2007; 370:1764-7). The results of these studies motivated the construction of large demonstrative trials in Italy, using hr-HPV DNA testing as a primary screening assay, followed by cytological examination of positive cases (Confortini M. et al. J Med Screen. 2010; 17:79-86). It is to be noted that not only CIN1 lesions are regressive, but also CIN2 and CIN3 (which correspond to HSIL in Pap test) often regress, especially in young women (Schiffman M. et al. **2007,** *loc. cit.;* Ronco G. et al. J Natl Cancer lnst, 2008; 100: 492-501).

The cited feature of screening programs implies that some percentage of over-diagnosis may be due to the screening test itself; however the over-diagnosis induced by cytological screening at three years intervals is considered to be acceptable. The Italian NTCC study (New Technologies for Cervical Cancer), started in 2004, has shown that the hr-HPV DNA test increases both over-diagnosis and over-treatment, compared to cytology in women aged less than 35 years, even if a cytological "triage" (that is to say a "diagnostic study" of the positive hr-HPV DNA test results) is carried out by Pap test (Ronco G. et G. et al. **2008,** *loc. cit.;* Ronco G. et al. Lancet Oncol 2010 Jan18). On the other hand, in women aged over 35 it was shown that the over-diagnosis, if it exists, is low even when a triage cytology is not adopted.

The above finding further highlights the need for specific biomarkers for high-grade CIN and cancer, intended for detecting molecular changes closely related with the transformation rather than the simple detection of hr-HPV DNA infections. The specificity is even more relevant when cohorts of women vaccinated against HPV will approach screening (Franco EL. et al. Vaccine. 2006; 24 Suppl 3:8171-7).

At present the only biomarkers ready to be used are those directly or indirectly related to the expression of the *E6* and *E7* viral genes, which regulate the replication of HPV. The *E6* and E7 genes from high-risk genotypes are known as oncogenes, and their altered transcriptional regulation, which affects almost all cellular pathways (Fehrmann F, Laimins LA. Oncogene. 2003; 22:5201-7) and promotes the instability of DNA, seems to be a necessary step for transformation of cells towards malignancy. The expression of HPV oncogenes and its impact on the host cell can be tracked directly, by identifying viral E6-E7 mRNA transcripts (Lie AK, Kristensen G., Expert Rev Mol Oiagn. 2008; 8:405-15), or indirectly through detection of the p16 cellular protein (Ca- rozzi F. et al., Lancet Oncol. 2008, 9:937-945; Benevolo M. et al. Am J Clin Pathol. 2008; 129:606-12). The latter, as is known, is a protein involved in cell cycle control, which is overexpressed in cervical cells transformed by HPV. In fact, as widely reported, p16 expression is influenced by hr-HPV E7 protein, and its up-regulation in the cervix is significantly related with increasing severity of the lesions (Benevolo M. et al,. Mod Pathol. 2006 Mar;19(3):384-9).

However, immunohistochemical evaluation of p16 currently presents a limited reproducibility due to lack of standard criteria for the interpretation of immunostaining (Tsoumpou I, et al., p16(INK4a) immunostaining in cytological and histological specimens from the uterine cervix: a systematic review and meta-analysis, Cancer Treat Rev. 2009; 35:210-20). Regarding E6-E7 mRNA of HPV could be a promising biomarker for the identification of clinically relevant hr-HPV DNA infections with a test entirely *in vitro.* Actually, it was seen that the E6-E7 mRNA increases with the severity of cervical disease (Sotlar K. et al., J Med Virol. 2004; 74:107-16; Castle PE. et al., Clin Cancer Res. 2007; 13:2599-605). It has been reported that both assays, p16 and E6-E7 mRNA, are a little less sensitive than hr-HPV DNA, but much more specific (Carozzi F. et al., Lancet Oncol. 2008, 9:937-945; Szarewski A. et al., Cancer Epidemiol Biomarkers Prev. 2008; 17:3033-4; Monsonego J. et al. EUROGIN 2010, Monaco. ES 4-4).

The line of research mentioned above (detection of the viral mRNA transcripts E6-E7) includes, for example, the European patents EP 1463839**,** EP 1718774 and EP 2267155 (Norchip A/S) and the US patent US 7524631 (Patterson).

So far, however, there are no biological markers that indicate the irreversible transformation of precancerous lesions towards cancer of the uterine cervix (Koo YJ. et al., Dual immunostaining of cervical cytology specimens with atypical squamous cells for p16/Ki-67 does not exclude the existence of a high-grade squamous intraepithelial lesion. Virchows Arch. 2013 Oct 1; Pacchiarotti A. et al., Prognostic value of p16-INK4A protein in women with negative or CIN1 histology result: A follow-up study. Int J Cancer. 2014, 134, 897-904).

Wang P et al., Chinese Journal of Experimental and Clinical Virology, vol. 14, n° 2, 1 June 2000, pages 117-120 and Wang P et al., Chinese Journal of Obstetrics and Gynecology, vol. 32, n° 12, 1 December 1997, pages 722-724, disclose methods for detecting a neoplastic cell transformation induced by human Papillomavirus (HPV), comprising detecting the presence of a protein complex E7/pRB.

### Summary of the invention

In the frame of the studies connected with the present invention, the interaction of the two viral oncogenes *E6* and *E7* with the oncosuppressor genes *p53* and *pRB* of the host cell has been considered. Actually, it is known that the oncogenic protein E6 binds to the oncosuppressive protein p53 of the host cell, inducing the degradation thereof and destroying the activity of stopping growth and activating apoptosis which is typical of this oncosuppressor. Similarly, the oncogenic protein E7 complexes and inactivates pRb protein of the host cell, thus nullifying its oncosuppressing activity (lshiji T., Molecular mechanism of carcinogenesis by human papillomavirus-16. J Dermatol 2000, Feb; 27(2):73-86L; Buitrago-Pérez A. et al., Molecular Signature of HPV-Induced Carcinogenesis: pRb, p53 and Gene Expression Profiling. Curr Genomics. 2009 Mar;10(1): 26-34; Shaikh F. et al., Molecular screening of compounds to the predicted Protein-Protein Interaction site of Rb1-E7 with p53-E6 in HPV. Bioinformation. 2012; 8(13): 607-12).

Specifically, expression of the *E6* and *E7* genes and the consequent production of related proteins would be the necessary cause to trigger HPV-induced oncogenesis, which is featured by the interactions between E6 and p53 proteins and E7 and pRb proteins respectively. E6 binds p53 in the cytoplasm and also recruits the E6AP ubiquitin ligase which makes p53 a target for proteasome degradation. Similarly, E7 binds pRb in the cytoplasm and recruits the Cullin2 ubiquitin ligase, which in turn promotes the degradation by the proteasome.

According to the present invention, it has been found that it is possible to identify by relatively simple examinations the very moment of carcinogenesis, by identifying the presence of those proteins which, deriving from the interaction of viral oncogenes *E6* and *E7* with tumor suppressor genes p53 and *pRB* of the host cell, represent an index of irreversible neoplastic transformation. To be able to fulfill its function in selecting cases in which the neoplastic transformation has taken place compared to those in which, despite the occurrence of lesions also of high degree, the carcinogenesis process would still be reversible, the examinations proposed according to the invention should be inserted in the diagnostic procedure of the programs of screening or early diagnosis of cervical cancer after a positive result of the hr-HPV DNA test and/or a cytological diagnosis of atypia of the squamous cells (ASC) or worse. Preferably, the proposed test should be performed after the execution of both hr-HPV DNA and Pap test with positive results.

The proposed procedure according to the invention innovates the clinical management of women who have had a diagnosis of high-grade dysplastic lesions of the uterine cervix, related to HPV infection. Essentially, it amounts to analyzing with specific technologies the proteins encoded by the viral oncogenes (E6 and E7) and those encoded by the host cell's tumor suppressor genes (p53 and pRb), categorizing and measuring them according to the phases of integration and/or interaction of the virus genome in the host. As noted, the specificity of the proposed procedure is to define the status of irreversibility of malignant transformation due to the interaction of viral proteins with human proteins.

The method of the invention is as defined in the appended claims.

In the implementation of the method proposed according to the invention, the search for proteins derived from the interaction of E6 with p53 (specifically, the protein complex E6/p53) and from the the interaction of E7 with pRb (specifically, the protein complex E7/pRB) can be performed by any analytical technique that allows to identify, in a simple but reliable way, the formation of one or both of the mentioned protein complexes in a sample of cells taken from the squamous-columnar junction of the uterine cervix of a patient under examination. More specifically, the analytical technique adopted should be capable of detecting in the sample the presence of the protein E6 or protein E7 in conjunction with the presence of a larger protein complex that contains, respectively, the protein E6 or the protein E7 in union with the p53 protein or the pRB protein. In practice, the two techniques which are suitable to achieve in the best way such goal are: A) the analytical method of Western Blot and B) the Sandwich Enzyme-Linked ImmunoSorbent Assay (Sandwich ELISA).
A) Western Blot: a new cervical sampling is carried out and the sample is stored in normal saline at 4-8°C. The latter then undergoes analysis according to the biochemical method known as Western Blot (o immunoblot). This method is used to identify a protein in a given sample of tissue homogenate or cell extract. It envisages centrifugation of the sample with separation of the supernatant and hence separation of proteins, previously heath-denatured, on the basis of their molecular mass, by electrophoresis on polyacrylamide gel in the presence of sodium dodecyl sulphate (SDS-PAGE). The proteins are then transferred from the gel onto a membrane, typically of nitrocellulose, where they are recognized and bound by specific antibodies.
B) Sandwich ELISA: a new cervical sampling in liquid phase (ThinPrep - Hologic, Inc., Marlborough, MA, or SurePath - BD Diagnostics, Burlington, NC) is carried out, if not already performed for the previous tests, in which case the residual sample is used. The sample is analyzed by means of the enzyme immunoassay Sandwich ELISA. This is a method of immunological analysis used in biochemistry to detect the presence of a substance by means of one or more antibodies, to one of which an enzyme is bound: depending on the detection of the protein of interest, the method implies the direct transfer of the cervical sample in the reaction plate, duly prepared with specific antibodies anti-protein E6 and anti-protein E7, and subsequently tested by other specific anti-p53 protein and anti-pRb protein antibodies for the detection of the interaction of the protein complexes of interest.

The methods Western Blot and Sandwich ELISA are carried out, ac- cording to the present invention, using specific monoclonal antibodies - antibodies anti-protein E6 and anti-protein E7 from strains of HPV of high oncogenic risk, antibodies anti-p53 protein, antibodies anti-pRb protein - commercially available. These methods resulted to be able not only to identify the presence of the four proteins of interest, but also to detect the possible inter- action state between E6 and p53 (E6/p53) and between E7 and pRb (E7/pRB), which represents the irreversibility point of the transformation of precancerous lesions towards the uterine cervix carcinoma.

The search for these proteins in women who have had a diagnosis of cytological atypia of the squamous cells (ASC) or worse, proposed according to the invention, allows to increase the diagnostic accuracy of hr-HPV DNA test and triage Pap test, increasing the overall sensitivity and specificity of screening. In particular, the introduction of this search will increase the PPV of the screening (positive predictive value, which is the ratio of the true positive subjects and the total of all positive subjects, both true and false), and to re- duce then the over-diagnosis and the over-treatment.

### Detailed description of the invention

According to its broadest embodiment, therefore, the present invention specifically provides a method for detecting a neoplastic cell transformation induced by human Papillomavirus (HPV) in women undergoing a screening for uterine cervix carcinoma, comprising detecting, in samples of cells taken from the squamous-columnar junction of the cervix epithelium of a patient being examined, the presence of a protein complex E6/p53 made by the protein E6 with the protein p53 and/or a protein complex E7/pRb made by the protein E7 with the protein pRb, wherein the detection of at least one of said two protein complexes shows that in the patient under examination the carcinogenesis has become irreversible, said detection being performed by the combined use of antibodies anti-protein E6 and antibodies anti-protein E7 and of antibodies anti-protein p53 and antibodies anti-protein pRb, preferably in Western Blot or Sandwich ELISA analytic techniques.

In accordance with the conventional technique, procedure A) using the Western Blot for the detection of interactions E6/E7 and p53/pRB consists of the following steps: (a) sample preparation (e.g., homogenization/extraction, centrifuging and boiling for denaturation of the proteins in sample buffer); (b) gel electrophoresis; (c) transfer onto membrane; (d) blocking of non specific sites of the membrane, usually with bovine albumin (BSA) or milk; (e) identification of the protein sought by incubation with antibodies (one primary and one which recognizes the antigen to which the secondary follows, conjugated to a detection system, which recognizes the primary); (f) detecting the binding antibody-antigen usually by colorimetric reaction or chemiluminescence.

Therefore, the present invention further specifically provides a method for the detection of a neoplastic cell transformation induced by human papillomavirus (HPV) in women undergoing in screening for uterine cervix carcinoma, comprising the following operations according to the Western Blot technique:
a) preparing a sample of cells taken from the squamous-columnar junction of the cervix epithelium of a patient being examined, and placing it in
   physiological solution;
b) extracting proteins from said sample and denaturizing the proteins obtained;
c) subjecting the so obtained denatured proteins to electrophoresis on polyacrylamide gel;
d) transferring the proteins obtained from the previous operation on a membrane and neutralizing the free sites of said membrane;
e) identifying said proteins by incubating them with at least the following antibodies:
   primary monoclonal antibody anti-protein E6;
   primary monoclonal antibody anti-protein E7;
   primary monoclonal antibody anti-protein p53; and
   primary monoclonal antibody anti-protein pRb;
   and then incubating with secondary anti-IgG antibodies conjugated with biotin or with a messenger enzyme such as alkaline phosphatase;
f) subjecting the products from the previous operation to colorimetric detection or to detection by chemiluminescence;
wherein the results of said detection which show a double reaction band for at least one of the proteins E6 (molecular weight 18 kDa) and E7 (molecular weight 16 kDa), said double band comprising a first band corresponding to the molecular weight of the protein sought, i.e. E6 or E7, and a second band corresponding to a molecular weight distinct and higher than the molecular weight of the respective protein of the first band, said molecular weight being substantially equal to 71 kD for the protein complex E6/p53 and to 115,5 kD for the protein complex E7/pRB, indicate that in the patient examined carcino-genesis has become irreversible; and/or wherein the results of said detection showing a double reaction band for at least one of the proteins p53 and pRb, said double band comprising a first band corresponding to the molecular weight of the protein sought, p53 or pRb, and a second band corresponding to a molecular weight distinct and higher than the molecular weight of the respective protein of the first band, indicate that in the patient examined carcinogenesis has become irreversible. The molecular weights corresponding to the second band, which is evident in these cases, are substantially equal to the sum of the molecular weights of p53 and E6, or E7 and pRb, thereby indicating a true interaction between the two proteins of the pair.

Preferably, according to the invention, those results of said detection according to step f) showing a double band of reaction, as described above, both for protein E6 and for protein E7, are considered, with greater security, indicative of a carcinogenesis already established. According to a preferred embodiment of the invention, in the phase e) previously cited proteins are also incubated with the following anti-bodies:
As noted, the interactions between the proteins E6 and p53 and between proteins E7 and pRb and cause, *de facto,* the degradation both of p53 and of pRb. Considering that p53 causes the cell cycle arrest in G1 phase, or it triggers the mechanisms of apoptosis, allowing the suppression of tumor cell clones and ensuring genomic stability, and that pRb is a protein with suppressor activity of cell growth, it understands how the interaction between E6 and p53 and E7 and pRb and the subsequent degradation produce *de facto* the abolition of the normal regulatory function of the two genes, which probably acts as a trigger for the neoplastic transformation of the cell.

According to the method of the invention, the results of said detection showing a single band of reaction for both proteins E6 and E7 (where the single band corresponding to the molecular weight of the protein sought, E6 or E7) indicate that in the patient under examination the carcinogenesis has not started or has not yet become irreversible. According to the same criterion, if the results of the detection do not show any reaction band for proteins E6 and E7, the results indicate that in the patient examined no integration of viral DNA with cellular DNA has occurred.

Preferably, according to the invention, the secondary antibodies anti-IgG of said step e) are conjugated with alkaline phosphatase, and the detection of step f) is a colorimetric or chemiluminescent detection. Furthermore, according to the preferred embodiments of the invention, the above mentioned primary monoclonal antibodies anti-protein E6 and anti-protein E7 are mouse monoclonal antibodies directed against the protein E6, or protein E7, of the viral type HPV16 and HPV18.

Also according to the preferred embodiments of the invention, the primary monoclonal antibody anti-p53 protein is a mouse monoclonal antibody anti-human p53 protein and the primary monoclonal antibody anti-pRb protein is a mouse monoclonal antibody anti-human pRb protein.

As noted earlier, the diagnostic method according to the invention is preferably inserted as part of a screening of a female population for the uterine cervix carcinoma, after a positive result of hr-HPV DNA test and/or after a cytological diagnosis of atypia of squamous cells (ASC) or worse.

According to a specific embodiment of the method of the invention, the proposed analysis provides for a process of removal of epithelium cells of the squamous-columnar junction of the uterine cervix, preferably by means of a tool known as "cytobrush" (a disposable brush tip for the taking of endocervical cells for cytological examination, having bristles arranged radially helicoid around its own axis and a length sloping towards the end, so as to have a conical overall shape). The sample thus obtained is stored in a physiological solution (1 ml) at 4°C; and the extraction of the proteins of interest and analysis with Western Blot technology are carried out.
The cell sediment obtained by centrifugation of the sample is washed twice with sodium chloride 0.9% by weight. After centrifugation at 5,000 rpm for 5 minutes, cells are harvested in lysis buffer and after the addition of pro- tease inhibitors are let under stirring for 30 minutes at 4°C. The lysate is then centrifuged for 20 minutes at 14,000 rpm at 4°C to extract the proteins from the supernatant. The proteins thus obtained are analyzed by electrophoresis on polyacrylamide gel in presence of sodium dodecyl sulfate, (SDS-PAGE) after heat denaturation (95°C for 5 minutes) in a specific buffer. The proteins are transferred to Poly Screen membrane (DuPont NEN).

The recovered gel and the membrane are immersed for 15 minutes in two separate trays containing the transfer buffer, preparing the sandwich by placing the components in sequence. For this aim, the system Semi-Dry-Electroblotter of aicos-Denmark may be used. As a confirmation of the transfer, the membrane is preferably colored with red Panceau in a pan on a shaking plate for 5 minutes, washing it with deionized water.

Again according to the aforementioned specific embodiment, the neutralization of the free sites of the membrane and subsequent analysis of the proteins of interest are carried out. The membrane prepared with a wash buffer containing 2% Milk is analyzed for the presence of protein using the following monoclonal primary antibodies for 90 minutes by continuous stirring:
- for protein E6, mouse primary monoclonal antibody anti protein E6 *(HPV16 E6* + *HPV18 E6 antibody [C1P5], Abcam);*
- for protein E7, mouse primary monoclonal antibody anti-protein E7 *(HPV16-E7 antibody [No. 28-0006], Invitrogen Corporation);*
- for protein p53, mouse primary monoclonal antibody anti-protein p53 *(Anti-p53 antibody [No. AH00112], Invitrogen Corporation);* e
- for protein pRb, mouse primary monoclonal antibody anti-protein Rb *(Anti-Human Rb antibody [No. 554162], BD Biosciences).*
The incubation of secondary mouse antibodies anti-IgG is carried out; then the washing and the development of the reactions by phosphatase are carried out, in order to detect the presence of viral oncoprotein E6 and E7 and their possible interaction respectively with proteins p53 and pRb, interpreting the results as illustrated.

In accordance with the conventional technique, the procedure B), that uses the Sandwich ELISA for detection of interactions E6 / E7 and p53 / pRB, following the scientific concepts relating to the invention already set out for the method A) of the Western Blot, consists of the following phases: (a) identifying the stored sample according to the liquid phase method (LBC) in use; (B) adsorbing in different wells of a microtiter plate capture antibodies of proteins E6 or E7; (C) blocking the remaining sites of the well binding the proteins by blocking buffer; (D) dispensing the sample directly from the LBC transport in each well; (E) identifying the proteins of interest by incubation with the diluted antibody anti-p53 protein in the wells adsorbed with antibody anti-protein E6 and the diluted antibody anti-pRb protein in the wells adsorbed with antibody anti-E7 protein; (F) blocking the endogenous substrate with a solution of 0.3% H202 in methanol; (G) incubating in their respective wells the antibody conjugated to horseradish peroxidase (HRP); (H) dispensing the chromogen solution TMB (3,3',5,5'-tetramethylbenzidine) in each well; (I) proceeding to the reading of the results by the quantization of bluish color with the spectrophotometer at a wavelength of 450 nm.
In order to validate the test, the above operations are performed in two separate wells that differ for the type of primary antibody which is introduced initially: one well is filled with a specific antibody for the protein (E6 or E7) that has to be identified, while the other is saturated with a non-specific antibody.
For the test to be considered valid, it firstly has to result negative in the latter well and in the case where the biological sample tested actually contains the antibody of interest, the test should be positive in the other well, the one saturated with the primary antibody specific for the protein of interest.

Therefore, the present invention further specifically concerns a method for the detection of a neoplastic cell transformation induced by human papillomavirus (HPV) in women undergoing screening for the uterine cervix carcinoma, comprising the following operations according to the Sandwich ELISA technique:
a) identifying a cell sample withdrawn from the squamous-columnar junction of the uterine cervix of a woman under examination, stored in solution according to the liquid phase cytology method (LBC) in use;
b) adsorbing, in different wells of the microplate in PVC the capture antibodies of the proteins E6 and E7 by incubating at 4°C overnight and subsequent washing with PBS (Phosfate Buffered Saline);
c) blocking the remaining sites of the well bonding the proteins by a blocking buffer containing no-fat dry milk in PBS, incubating at least 1-2 hours at room temperature or overnight at 4°C;
d) dispensing 100 microliters of sample from the liquid phase cytology bottle directly in each well, incubating at 37°C for 90 minutes and removing by a further washing in PBS;
e) identifying the proteins of interest by incubation with 100 microliters of diluted antibody anti-protein p53 in the wells adsorbed with antibody anti-protein E6 and 100 microliters of diluted antibody anti-protein pRb in the wells adsorbed with antibody anti-protein E7, at room temperature for 1-2 hours and removing with further repeated washing in PBS;
f) blocking the endogenous substrate by incubation a room temperature for 20 minutes with 0,3% solution of H₂O₂ in methanol;
g) incubating into the respective wells 100 microliters of a solution containing the antibody conjugated to horseradish peroxidase (HRP);
h) dispensing a chromogen solution TMB in each well, for 20 minutes (enzymatic reaction: H₂O₂ + reduced substrate 2 H₂O + oxidized substrate) and blocking the reaction by addition of a solution 2M of H₂O₂;
i) subjecting the step h) reaction product to reading of the light intensity detected at 450 nm wavelength, by spectrophotometer,
wherein results showing the occurred staining, documented by a positive absorbance, indicate that in the patient under examination protein complex E6/p53 and/or E7/pRB are present, confirming interaction between viral proteins and those human and therefore the presence of irreversible carcinogenesis.

The control of the test is validated in two separate wells that differ for the type of primary antibody introduced initially: one well is filled with a specific antibody for the protein (E6 or E7) that wants to search, least-three the other is saturated with a non-specific antibody. The test is considered valid if it is negative in the last cockpit and positive in the cockpit, that is saturated with the primary antibody specific for the protein of interest.

According to the method of the invention, for the steps e), f), g) and h) it is possible to use other systems available on the market that allow to amplify the chromogenic signal (for example, using antibodies anti-p53 or pRb protein biotinylated and highlighted with streptavidin conjugated to peroxidase or to
another enzyme).
Preferably, according to the invention, results that, with greater security, are considered indicative of an already established carcinogenesis are those results of said detection according to step i) showing absorbance values detected for both the complex E6/p53 for the complex E7/pRb.

According to the method of the invention, the results of this detection showing a positive reaction for both protein complexes (E6/p53 and E7/pRb) indicate that in the patient under examination the carcinogenesis has already started and become irreversible. According to the same criterion, in the case where the detection results not showing any positivity of the reaction (i.e. absence of absorbance signal) for both protein complexes (E6/p53 and E7/pRb), the result indicates that in the patient under examination the interaction of the viral DNA with cellular DNA has not occurred; not excluding the possibility of being in the presence of a viral DNA integration with the cell DNA not diagnosable with this procedure.

Preferably, according to the invention, the antibodies of said step e) are evidenced by reaction with a specific antibody conjugated to horseradish peroxidase and the detection of the phases f), g) and h) is a detection of the colorimetric type. Furthermore, according to the preferred embodiments of the invention, the above mentioned primary monoclonal antibodies anti-E6 protein and anti-E7 protein are mouse monoclonal antibodies directed against the protein E6, or the E7 protein, of the viral types HPV16 and HPV18.

Also according to the preferred embodiments, the primary monoclonal antibody anti-p53 is a mouse monoclonal antibody anti-human p53 protein and the primary monoclonal antibody anti-pRb protein is a mouse monoclonal antibody anti-human pRb protein.

As noted earlier, the diagnostic method according to the invention is preferably inserted as part of a screening of a female population for the uterine cervix carcinoma, after a positive test result hr-HPV DNA and/or after a cytological diagnosis of atypic squamous cell (ASC) or worse.
According to a specific embodiment of the method of the invention, the proposed analysis provides for a process of removal of epithelium cells of the squamous-columnar junction of the uterine cervix, preferably by means of a tool known as "cytobrush". The sample thus obtained is preserved in a solution for in liquid phase cytology and then the analysis of proteins of interest is carried out with Sandwich ELISA technology.

Still according to the aforementioned specific embodiment, the cells contained in the sample for liquid phase cytology are tested directly, without proceeding with the extraction of proteins, by sandwich antibody reaction: a viral antibody anti-protein (E6 or E7) adsorbed on the plate, protein of interest complex (E6/E7 or p53/pRb) present in the sample, anti-human (p53 or pRb) protein. The method ensures the absolute binding specificity and documents the presence of the protein complexes E6/p53 and E7/pRb by the use of the following primary monoclonal antibodies:
- for protein E6, mouse primary monoclonal antibody anti protein E6 *(HPV16 E6* + *HPV18 E6 antibody [C1P5], Abcam)*;
- for protein E7, mouse primary monoclonal antibody anti-protein E7 *(HPV16-E7 antibody [No. 28-0006], Invitrogen Corporation)*;
- for protein p53, mouse primary monoclonal antibody anti-protein p53 *(Anti-p53 antibody [No. AHO0112], Invitrogen Corporation);* e
- for protein pRb, mouse primary monoclonal antibody anti-protein Rb *(Anti-Human Rb antibody [No. 554162], BD Biosciences).*
The subsequent incubation with mouse antibodies anti-IgG conjugated with peroxidase and the development of the colorimetric reaction documented by the spectrophotometric reading, confirm the positivity of the research of protein of interest complex interpreting the results as already illustrated.

The present invention further specifically provides, more generally, a model of diagnostic method for the detection of any neoplastic cell transformation induced by the interaction of any protein with oncogenic action (for example: BRAF, KRAS, NRAS, HER2, etc.) with another protein linked to the cell tumor suppressor action (e.g. p53, pRb, PTEN, BRCA, CD95, etc.) invalved in the various organs and systems of the body.

In particular, these proteins with oncogenic action or tumor suppressor activity are identified with specific monoclonal antibodies and their interaction is proven by Western Blot and/or ELISA Sandwich technologies. Preferably, according to the proposed methods, the protein complex that has interacted (oncogenic protein/tumor suppressor protein) is also identified directly with a specific monoclonal antibody.

As a whole, therefore, the invention allows to propose a method valid for the detection of any neoplastic cell transformation induced by the interaction of any protein with oncogenic activity with another protein linked to cellular tumor suppressor activity, according to as hereinbefore described, wherein said method is also inserted as part of a diagnostic program and/or therapeutic treatment of cancer patients, or patients suspected, that uses targeted therapies that inhibit said protein interaction.

### Brief description of the drawings

The specific features of the invention, as well as the advantages thereof, will become more apparent referring to the description of the experimental work reported in the following with merely illustrative purposes and to the related figures, wherein:
**Figure 1** shows a histogram with the cytological diagnosis of 78 cases selected as positive from a population of 2500 women undergoing a screening with Pap-test.
**Figure 2** shows a histogram with the results of the application of the diagnostic method according to the present invention to the 78 cases selected as positive to the Pap-test of Figure 1;
**Figures 3-6** show the results of Western Blot of one case selected from the results of the diagnostic method according to the invention wherein E6 and E7 protein are present, and wherein E6/p53 and E7/pRb interactions are also present;
**Figure 7** is a flow diagram schematically illustrating the indications proposed for women management with positive Pap test and positive hr-HPV DNA test for the application of the diagnostic method of the invention; and

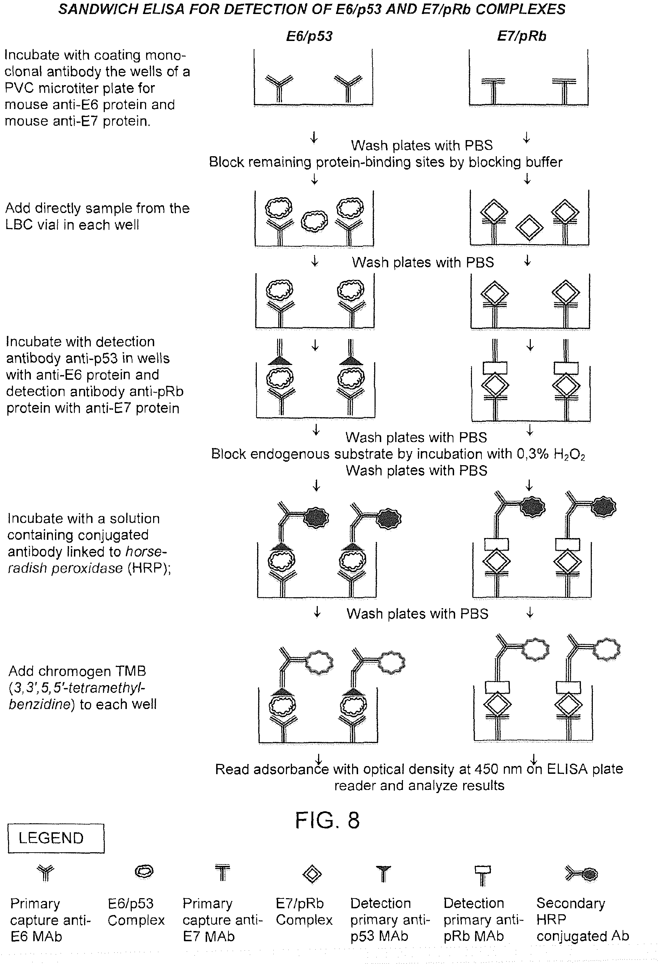

**Figure 8** is a flow diagram schematically illustrating the application of the Sandwich ELISA method to the diagnostic method of the invention.

### Screening test on a population of women not selected undergoing Pap test as primary examination

In the context of the present invention, a study was carried out aiming at identifying women with clinically relevant lesions that had developed high grade dysplastic lesions or cervical cancer. The study was conducted on 2,500 cervical specimens of women, not selected, which occurred spontaneously at health advisory outpatient clinics centers of Caserta Local Health Board ASL. The research was carried out in the period from June to September 2012 and included information to women and the acquisition of informed consent. In this population conventional Pap tests have been performed and the tests have been analyzed at the Cytopathology Laboratory of Caserta ASL.

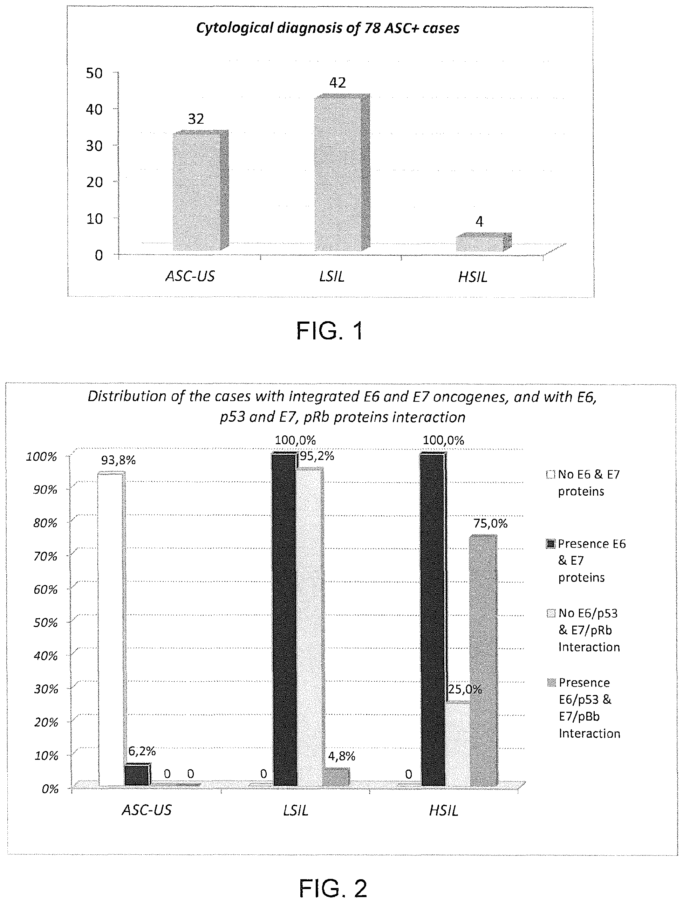

Of the 2,500 women surveyed, a total of 78, that is, 3.12%, were diagnosed not negative, that is, ASC + (Squamous Cells Atypia or worse) to the Pap test. As also shown in the diagram of attached Fig. 1, the cytological diagnoses ASC +were divided as follows: 32 cases were with ASC-US cytology (41%), 42 cases were LSIL (54%) and 4 cases were HSIL (5%).

96.88% of cases were negative for squamous intraepithelial lesions or malignant ending in the categories of normal or benign inflammatory changes, sometimes marked by the presence of microorganisms (bacteria, fungi, or trichomonas vaginalis).

78 women with positive diagnosis were recalled to undergo a further examination according the method of the present invention, by a second sample to study the integration/interaction of E6/p53 and E7/pRb proteins.

Sample have been withdrawn and tested according to the procedure described above for the specific embodiment of the method according to the invention, at the Molecular Biology Laboratory of the Marcianise Hospital.
Data obtained by Western Blot analysis of the 78 samples positive Pap test are shown in the herein attached **Figure 2****.** The histogram firstly shows that of the 32 samples with ASC-US cytological diagnosis only 2 (6.3%) were positive for presence of while in the other 30 (93.7%) E6 and E7 proteins were not found.

On the other hand, in all samples with a cytological diagnosis of LSIL (42) and HSIL (4) has been proved the presence of E6 and E7 proteins. In total, therefore, of the 78 cases which have had a not negative diagnosis with Pap tests, only 48 were positive for the presence of the E6 and E7 proteins according to the diagnostic method according to the invention.

From the data observation, it is clear that in most of ASC-US (93.7%) integration of viral genome did not occur, and as result it may mean:
*1) HPV absent: cytological abnormalities found are only inflammatory in nature;*
*2) low-risk HPV present: normally is not able to integrate into genome but is limited to the episomal form;*
*3) high-risk HPV present: it is still in the early stage with very recent infection and lack of integration.*

For all cases with LSIL and HSIL cytological diagnosis were identified E6 and E7 proteins and therefore it can be stated that these samples have a high-risk HPV and certainly the infection is not recent, as already had the viral genome integration.
Taking in consideration the 48 positive cases with the presence of proteins E6 and E7, also as shown in **Figure 2****,** the diagnostic method of the pre- sent invention allows to observe that the interactions E6/p53 and E7/pRb are present in 4,8% of the LSIL cases and in 75,0% of the HSIL cases, being absent in the ASC-US showing the presence of proteins E6 and E7.
The results shown in the histogram of **Figure 2** are also summarized in the following table.

**TABLE 1**

| Correlation of cytological and histological diagnosis of the cases tested with Western Blot for integration/interaction of proteins E6/p53 and E7/pRb. | | | | | |
|---|---|---|---|---|---|
| **Cytological diagnosis** | **Presence of E6 & E7 proteins** | **No E6/p53 & E7/pRb interaction** | | **Presence of E6/p53** & **E7/pRb interaction** | |
| | | | *Histology* | | *Histology* |
| ASC-US | 2 | 2 (100,0%) | 2 CIN1-2 | 0 | |
| LSIL | 42 | 40 (95,2%) | 37 CIN1 | 2 (4,8%) | 1 CIN2 |
| | | | 3 CIN2 | | 1 CIN3 |
| HSIL | 4 | 1 (25,0%) | 1 CIN3 | 3 (75,0%) | 2 CIN3 |
| | | | | | 1 CA. SQ. INV. |
| TOTAL | 48 | 43 (89,6%) | 37 CIN1 | 5 (10,4%) | 1 CA. SQ. INV. |
| | | | 2 CIN1-2 | | 3 CIN3 |
| | | | 3 CIN2 | | 1 CIN2 |
| | | | 1 CIN3 | | |

The data shown above demonstrate that 48.8% of LSIL and 75.0% of HSIL have the interaction of E6/p53 and E7/pRb leaving to assume that car- cinogenesis process is at advanced stage; for remaining LSIL and HSIL cases without interaction, it can be assumed that the carcinogenesis process is still in early stages and still regression is possible.

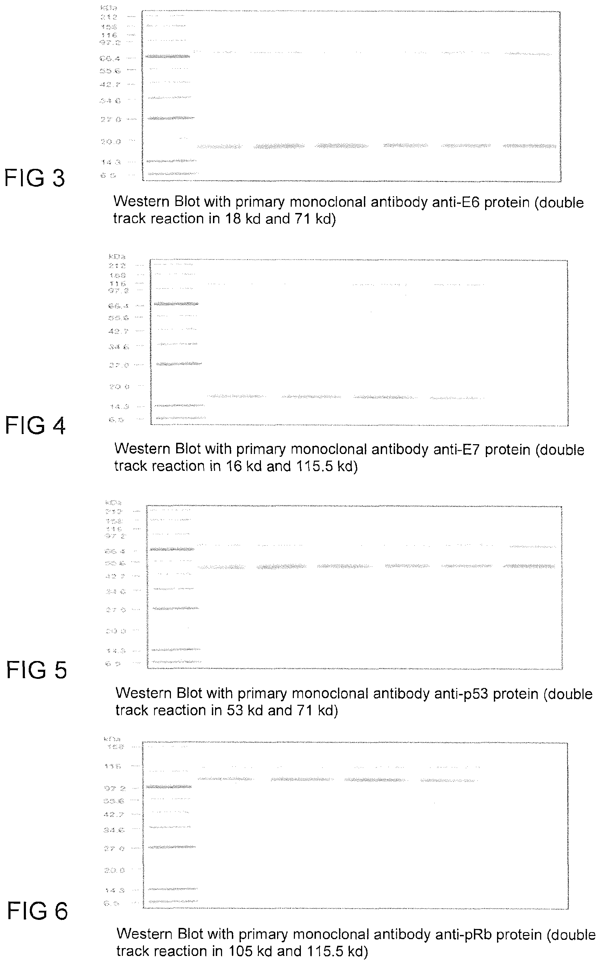

The most significant examples of the type of results obtained by West- ern Blot in the study herein reported are shown in the attached **Figures 3, 4, 5** and **6****,** which show the results of Western Blot analysis obtained from samples where in addition to E6 and E7 was also found interaction with p53 and pRb.

The observation of **Figures. 3** and **4****,** respectively relating to the Western Blot results with primary monoclonal antibody anti-E6 and anti-E7 protein shows a double stripes of reaction, for each sample, 18 Kd and 71 Kd for anti-E6 protein, 16 Kd and 115.5 Kd for anti-E7 protein 7. The existence of additional stripes of molecular weight much higher those E6 and E7 demonstrates, according to the present invention, the presence of an interaction between E6 and p53 and between E7 and pRb.

Said hypothesis has been confirmed by two additional Western Blot analysis with primary monoclonal antibodies, respectively, anti-p53 and anti-pRb protein. In **Figures 5** and **6** are shown the results obtained which confirm such hypothesis, as the reactive stripes exactly match the two additional stripes previously observed.

For samples with the E6 and E7 proteins but without interaction E6/p53 and E7/pRb, the Western Blot with primary monoclonal antibody anti-E6 protein and anti-E7 protein have shown, for each sample, a single reactive stripe of respectively 18 Kd for the antibody anti-E6 protein and 16 Kd for anti-E7 protein.

For the samples, however, which were negative for the presence of E6 and E7 proteins, both the Western Blot with primary monoclonal antibody anti-E6 protein that with anti-E7 protein showed no reaction stripe, evidence that in these samples there has been no integration of viral DNA into the cell.

According to the results obtained, it is clear that all HSIL and majority of LSIL studied present the integration of the viral genome with E6 and E7 proteins production. However, 75.0% of high-grade lesions and only few low- grade lesions (4.8%) showed the interaction of proteins E6/p53 and E7/pRb. These data confirm that the interaction is a marker of carcinogenesis.
Therefore, the Western Blot technique for assessing the presence of E6 and E7 proteins and their possible interaction (E6/p53 and E7/pRb) demonstrates a higher specificity of both HPV DNA test and cytology, allowing identifying patients with clinically relevant lesions (CIN2+). Histological follow-up of LSIL and HSIL have confirmed this hypothesis. In particular (see, Table 1), the four HSIL who had E6/p53 and E7/pRb proteins interaction, two were CIN 3 (Severe Dysplasia - Carcinoma in situ) (50.0%) and one invasive squamous cell Carcinoma (25.0%), the fourth case without interaction was diagnosed CIN 3. Also the two LSIL (4.8%) with E6/p53 and E7/pRb proteins interaction are findings one CIN2 (Moderate Dysplasia) (50.0%) and the other CIN 3 (50.0%). The 40 LSIL (95.2%) without interaction were 37 CIN 1 (Mild dysplasia) (88.1%) and 3 CIN2 (7.1%).
These information confirm that there is a relationship between protein interactions and carcinogenesis, even it is still to understand what are biological bases of step from integrated with those interactive and thus to carcinomatous transformation. Basically only few integrated lesions expressing E6 and E7 proteins overexpression progressing toward complete carcinogenesis. This suggests the hypothesis that there is a quantitative threshold of viral proteins over which their interaction develops. Further studies will be able to confirm this hypothesis according to the most representative case studies.

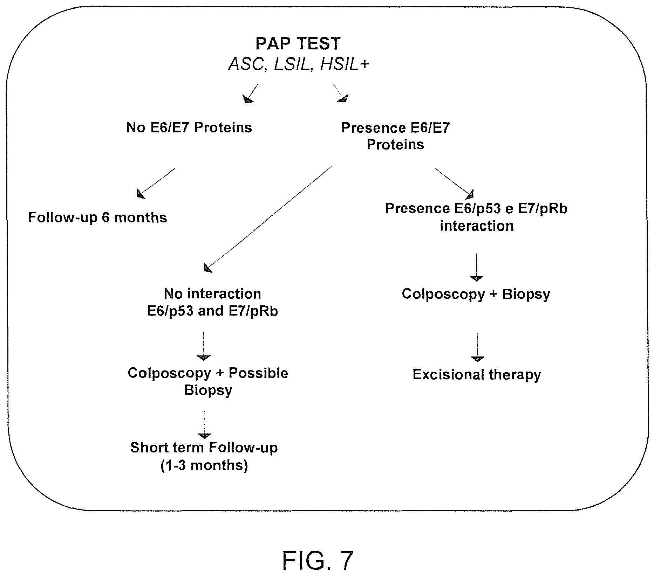

Considering the evaluation above reported as fundamental, according to the method of the invention, we propose Western Blot or Sandwich ELISA for carrying out the *integration*/*interaction test of proteins E6*/*p53 and E7*/*pRb* in diagnostic pathway after hr-HPV DNA positive test and Pap test positive for ASC+ lesions, in particular according the schematic indications in the diagram of **Figure 7****.**

Using this diagnostic strategy it is possible increase the specificity of cytology (ASC+) and molecular (hr-HPV DNA positive test) diagnosis in cervical screening, reserving further recall women to the second level of investigation just the cases with integration/interaction of viral genome. The same strategy may be applied also for neoplastic lesions HPV-induced in extra-uterine areas (anus-genital region, oropharyngeal region, etc.).

An efficient vaccine strategy combined with organized screening and adequate sensitivity and increasing specificity of used tests, as the one here reported as an example, will lead to the decrease of papillomavirus infection prevalence by resulting in decreased impact of cervical cancer, health and socio-economic advantage for society, greatly reducing mortality, also by targeted therapy.

A further possible advantageous use of the method according to the invention consists in the use of the same method for making experiments, in the various phases of the disease progression, about the activities of anticancer drugs and the inhibiting activity of the interaction E6/p53 and E7/pRb as nicandrenone (Shaikh F. et al. **2012,** loc. cit.), for the develop and experiment of said drug in targeted therapy.
The same methods are to be intended also to identify, in cells samples taken from any anatomical site other than the uterine cervix of any patient un- der examination, the proteins encoded by oncogenic genes, also non-viral, and those encoded by tumor suppressor genes in the cell examination and to determine, by Western Blot and/or ELISA Sandwich, the possible interaction between them.

## Claims

1. A method for detecting a neoplastic cell transformation induced by human Papillomavirus (HPV) in women undergoing a screening for uterine cervix carcinoma, comprising detecting, in samples of cells taken from the squamous-columnar junction of the cervix epithelium of a patient being examined, the presence of a protein complex E6/p53 made by the protein E6 with the protein p53 and a protein complex E7/pRb made by the protein E7 with the protein pRb, wherein the detection of said two protein complexes shows that in the patient under examination the carcinogenesis has become irreversible, said detection being performed by the combined use of antibodies anti-protein E6, antibodies anti-protein E7, antibodies anti-protein p53 and antibodies anti-protein pRb.

2. The method according to claim 1, comprising the following steps according to the Western Blot technique:
a) preparing a sample of cells taken from the squamous-columnar junction of the cervix epithelium of a patient being examined, and placing it in physiological solution;
b) extracting proteins from said sample and denaturizing the proteins obtained;
c) subjecting the so obtained denatured proteins to electrophoresis on polyacrylamide gel;
d) transferring the proteins obtained from the previous step on a membrane and neutralizing the free sites of said membrane;
e) identifying said proteins by incubating them with at least the following antibodies:
primary monoclonal antibody anti-protein E6;
primary monoclonal antibody anti-protein E7;
primary monoclonal antibody anti-protein p53;
primary monoclonal antibody anti-protein pRb;
and then incubating with secondary anti-IgG antibodies conjugated with biotin or with a messenger enzyme such as alkaline phosphatase;
f) subjecting the products from the previous step to colorimetric detection or to detection by chemiluminescence;
wherein the results of said detection showing a double reaction band for the proteins E6 and E7, said double band comprising a first band corresponding to the molecular weight of the protein sought, i.e. E6 or E7, and a second band corresponding to a molecular weight distinct and higher than the molecular weight of the respective protein of the first band, said molecular weight being substantially equal to 71 kD for the protein complex E6/p53 and to 115,5 kD for the protein complex E7/pRb, indicate that in the patient examined carcinogenesis has become irreversible; and/or the results of said detection showing a double reaction band for at least one of the proteins p53 and pRb, said double band comprising a first band corresponding to the molecular weight of the protein sought, p53 or pRb, and a second band corresponding to a molecular weight distinct and higher than the molecular weight of the respective protein of the first band, indicate that in the patient examined carcinogenesis has become irreversible.

3. The method according to claim 2, wherein the results of said detection showing a single reaction band for both proteins E6 and E7, said single band corresponding to the molecular weight of the protein sought E6 or E7, indicate that in the patient examined carcinogenesis is not yet started and has not become irreversible.

4. The method according to claim 3, wherein the results of said detection which do not show any reaction band for proteins E6 and E7 indicate that in the patient examined no integration of viral DNA with cellular DNA has occurred.

5. The method according to claim 2, wherein the secondary anti-IgG antibodies of said step e) are conjugated with alkaline phosphatase, and wherein said detection of operation f) is a colorimetric detection or a detection by chemiluminescence.

6. The method according to claim 2 wherein said primary monoclonal antibody anti-protein E6 is a mouse anti-protein E6 antibody of the viral types HPV16 and HPV18, and said primary monoclonal antibody anti-protein E7 is a mouse anti-protein E7 antibody of the viral types HPV16 and HPV18.

7. The method according to claim 2, wherein said primary monoclonal antibody anti-protein p53 is a mouse monoclonal anti-human protein p53 antibody, and said primary monoclonal antibody anti-protein pRb is a mouse monoclonal anti-human protein pRb antibody.

8. The method according to claim 1, wherein said detection is performed by the combined use of antibodies anti-protein E6, antibodies anti-protein E7, antibodies anti-protein p53 and antibodies anti-protein pRB, comprising the following steps according to the Sandwich ELISA technique:
a) identifying a sample to be examined, stored according to liquid phase cytology (LBC);
b) adsorbing, in different wells of a microplate, antibodies anti-protein E6 and anti-protein E7, as capture anti-bodies of viral protein E6 or E7;
c) blocking the remaining sites of the well bonding the proteins by a blocking buffer;
d) dispensing directly into each well the indicated cell sample to be analyzed;
e) identifying the proteins of interest by incubation with diluted antibody anti-protein p53 in the wells adsorbed with antibody anti-protein E6 and by diluted antibody anti-protein pRb in the wells adsorbed with antibody anti-protein E7;
f) blocking the endogenous substrate;
g) incubating into the respective wells the antibody conjugated to horseradish peroxidase (HRP);
h) dispensing a chromogen solution in each well
i) subjecting the step h) reaction product to reading of the light intensity detected at 450 nm wavelength, by spectrophotometer,
wherein results showing the occurred staining, documented by a positive absorbance, indicate that in the patient under examination the protein complexes E6/p53 and E7/pRb are present, confirming interaction between viral and human proteins, and therefore indicating that in the patient under examination the carcinogenesis started and has become irreversible.

9. A method according to of claim 8, wherein in steps e), f), g) and h), biotinylated anti-protein p53 and pRB antibodies are used and detected with streptavidin conjugated to peroxidase or to another enzyme to amplify the chromo- genie signal.

10. The method according to claim 8, wherein in case the detection results do not show any reaction positivity, with absence of signal absorbance at 450 nm, for both protein complexes E6/p53 and E7/pRb, the result indicates that in the patient under examination no integration of viral DNA with cellular DNA has occurred; not excluding the possibility of being in presence of an integration of viral DNA with the cell DNA.

11. The method according to claim 8, wherein said primary monoclonal antibody anti-protein E6 is a mouse monoclonal antibody anti-protein E6 of the viral types HPV16 and HPV18, said primary monoclonal antibody anti-protein E7 is a mouse monoclonal antibody anti-protein E7 of the viral type HPV16, said primary monoclonal antibody anti-protein p53 is a mouse monoclonal antibody anti-human p53 protein, said primary monoclonal antibody anti-pRb protein is a·mouse monoclonal antibody anti-human pRb protein.

12. The method for detecting a neoplastic cell transformation induced by human Papillomavirus (HPV) according to any one of claims 2-5 and 9-12, wherein said method is inserted in the frame of a screening program of a female population, or in a program for early diagnosis, for the detection of uterine cervix carcinoma, after a cytological diagnosis of atypical squamous cells (ASC) or worse, and a subsequent positive result of the hr-HPV DNA test.

13. The method for detecting a neoplastic cell transformation induced by human Papillomavirus (HPV) according to any one of claims 2-5 and 9-12, wherein said method is inserted in the frame of a screening program of a female population, or in a program for early diagnosis, for the detection of uterine cervix carcinoma, after a positive result of the hr-HPV DNA test, and sub- sequent cytological diagnosis of atypical squamous cells (ASC) or worse.

14. The method for detecting a neoplastic cell transformation induced by human Papillomavirus (HPV) according to any one of claims 2-5 and 9-12, wherein said method is inserted in the frame of diagnostic activity, screening or follow-up of patients with neoplastic lesions HPV induced, or suspected such, in extra-uterine areas, as the anus-genital region, oropharyngeal region.

## Patentansprüche

1. Verfahren zum Nachweisen einer neoplastischen Zellumwandlung, die durch humane Papillomaviren (HPV) in Frauen induziert wird, welche sich einer Vorsorgeuntersuchung für Gebärmutterhalskrebs unterziehen, umfassend den Nachweis, in Zellproben, die aus der Platten-Zylinder-Verbindung des Gebärmutterhalsepithels eines zu untersuchenden Patienten entnommen sind, für das Vorliegen eines Proteinkomplexes E6/p53, der durch das Protein E6 mit dem Protein p53 hergestellt wird, und eines Proteinkomplexes E7/pRb, der durch das Protein E7 mit dem Protein pRb hergestellt wird, wobei der Nachweis der zwei Proteinkomplexe zeigt, dass in dem zu untersuchenden Patienten die Krebsentstehung irreversibel geworden ist, und der Nachweis durch die kombinierte Verwendung von Anti-Protein E6 Antikörpern, Anti-Protein E7 Antikörpern, Anti-Protein p53 Antikörpern und Anti-Protein pRb Antikörpern durchgeführt wird.

2. Verfahren nach Anspruch 1, umfassend die folgenden Schritte gemäß der Western-Blot-Technik:
a) Herstellen einer Zellprobe, die aus der Platten-Zylinder-Verbindung des Gebärmutterhalsepithels eines zu untersuchenden Patienten entnommen wird, und deren Anordnen in einer physiologischen Lösung;
b) Extrahieren von Proteinen aus der Probe und Denaturieren der erhaltenen Proteine;
c) Unterziehen der so erhaltenen denaturierten Proteine einer Elektrophorese auf einem Polyacrylamidgel;
d) Überführen der aus dem vorhergehenden Schritt erhaltenen Proteine auf eine Membran und Neutralisieren der Leerstellen auf der Membran;
e) Identifizieren der Proteine durch deren Inkubieren mit zumindest den folgenden Antikörpern:
primären monoklonalen Anti-Protein E6 Antikörpern;
primären monoklonalen Antikörper Anti-Protein E7 Antikörpern;
primären monoklonalen Antikörper Anti-Protein p53 Antikörpern;
primären monoklonalen Antikörper Anti-Protein pRb Antikörpern;
und nachfolgendes Inkubieren mit sekundären Anti-IgG Antikörpern, die mit Biotin oder mit einem Messenger-Enzym, wie alkalischer Phosphatase, konjugiert sind,
f) Unterziehen der Produkte aus dem vorherigen Schritt einem kolorimetrischen Nachweis oder einem Nachweis durch Chemilumineszenz;
wobei die Ergebnisse des Nachweises, welche eine Reaktionsdoppelbande für die Proteine E6 und E7 zeigen, und die Doppelbande eine erste Bande entsprechend dem Molekulargewicht des gesuchten Proteins, d. h. E6 oder E7, und eine zweite Bande entsprechend einem Molekulargewicht, welches verschieden und höher ist als das Molekulargewicht des entsprechenden Proteins der ersten Bande, umfasst, und das Molekulargewicht im Wesentlichen 71 kD für den Proteinkomplex E6/p53 und 115,5 kD für den Proteinkomplex E7/pRb entspricht, anzeigen, dass in dem zu untersuchenden Patienten eine Krebsentstehung irreversibel geworden ist; und/oder die Ergebnisse des Nachweises eine Reaktionsdoppelbande für zumindest eines der Proteine p53 und pRb zeigen, und die Doppelbande, eine erste Bande entsprechend dem Molekulargewicht des gesuchten Proteins, p53 oder pRb, und eine zweite Bande entsprechend einem Molekulargewicht, welches verschieden und höher ist als das Molekulargewicht des entsprechenden Proteins der ersten Bande, umfasst, anzeigen, dass in dem zu untersuchenden Patienten eine Krebsentstehung irreversibel geworden ist.

3. Verfahren nach Anspruch 2, wobei die Ergebnisse des Nachweises eine einzelne Reaktionsbande für beide Proteine E6 und E7 zeigen, und die Einzelbande dem Molekulargewicht des gesuchten Proteins E6 oder E7 entspricht, zeigen, dass in dem zu untersuchenden Patienten eine Krebsentstehung noch nicht begonnen hat und noch nicht irreversibel geworden ist.

4. Verfahren nach Anspruch 3, wobei die Ergebnisse des Nachweises, welche keine Reaktionsbande für die Proteine E6 und E7 zeigen, anzeigen, dass in dem zu untersuchenden Patienten keine Aufnahme von viraler DNA in zelluläre DNA stattgefunden hat.

5. Verfahren nach Anspruch 2, wobei die sekundären Anti-IgG Antikörper aus Schritt e) mit alkalischer Phosphatase konjugiert werden, und wobei der Nachweis des Arbeitsschrittes f) ein kolorimetrischer Nachweis oder ein Nachweis durch Chemilumineszenz ist.

6. Verfahren nach Anspruch 2, wobei der primäre monoklonale Anti-Protein E6 Antikörper ein Anti-Protein E6 Antikörper der Maus der viralen Typen HPV16 und HPV18 ist, und der primäre monoklonale Anti-Protein E7 Antikörper ein Anti-Protein E7 Antikörper der Maus der viralen Typen HPV16 und HPV18 ist.

7. Verfahren nach Anspruch 2, wobei der primäre monoklonale Anti-Protein p53 Antikörper ein monoklonaler Anti-human-Protein p53 Antikörper der Maus ist, und der primäre monoklonale Anti-Protein pRb Antikörper ein monoklonaler Anti-human-Protein pRb Antikörper der Maus ist.

8. Verfahren nach Anspruch 1, wobei der Nachweis durch die kombinierte Verwendung von Anti-Protein E6 Antikörpern, Anti-Protein E7 Antikörpern, Anti-Protein p53 Antikörpern und Anti-Protein pRb Antikörpern durchgeführt wird, umfassend die folgenden Schritte gemäß der Sandwich-ELISA-Technik:
a) Identifizieren einer zu untersuchenden Probe, die entsprechend einer Flüssig-Phase-Zytologie (LBC) gelagert wird;
b) Adsorbieren, in verschiedenen Löchern einer Mikroplatte, von Antikörpern zu Anti-Protein E6 und Anti-Protein E7, als Bindungs-Antikörper von viralem Protein E6 oder E7;
c) Blockieren der verbleibenden Stellen des Loches, welche die Proteine binden, durch einen Blockierungspuffer;
d) direktes Ausgeben der zu untersuchenden angezeigten Zellprobe in jedes Loch;
e) Identifizieren der Proteine von Interesse durch Inkubieren mit verdünntem Antikörper Anti-Protein p53 in den Löchern, welche mit Antikörper Anti-Protein E6 adsorbiert sind, und durch verdünnte Antikörper Anti-Protein pRb in den Löchern, welche mit Antikörper Anti-Protein E7 adsorbiert sind;
f) Blockieren des endogenen Substrats;
g) Inkubieren der Antikörper, die zu Meerrettich-Peroxidase (HRP) konjugiert sind, in die jeweiligen Löcher;
h) Ausgeben einer chromogenen Lösung in jedes Loch;
i) Unterziehen des Reaktionsproduktes aus Schritt h) einer Erfassung der bei 450 nm Wellenlänge ermittelten Lichtintensität durch ein Spektralfotometer,
wobei die Ergebnisse, die die aufgetretene Fleckenbildung zeigen, und die durch eine positive Absorption dokumentiert ist, anzeigen, dass in dem Patienten, der untersucht wird, die Proteinkomplexe E6/p53 und E7/pRb vorliegen und eine Wechselwirkung zwischen viralen und humanen Proteinen bestätigen, und dadurch anzeigen, dass in dem Patienten, der untersucht wird, die Krebsentstehung begonnen hat und irreversibel geworden ist.

9. Verfahren nach Anspruch 8, wobei in den Schritten e), f), g) und h) biotinylierte Anti-Protein p53 und pRb Antikörper verwendet werden, und mit Streptavidin nachgewiesen wird, welches mit Peroxidase oder einem anderen Enzym konjugiert ist, um das chromogene Signal zu verstärken.

10. Verfahren nach Anspruch 8, wobei für den Fall, dass die Nachweisergebnisse bei einer Abwesenheit einer Signalabsorption bei 450 nm für beide Proteinkomplexe E6/p53 und E7/pRb keine positive Reaktion zeigen, das Ergebnis anzeigt, dass in dem Patienten, der untersucht wird, keine Aufnahme von viraler DNA in die zelluläre DNA aufgetreten ist; ohne die Möglichkeit des Vorliegens einer Aufnahme von viraler DNA in die zelluläre DNA auszuschließen.

11. Verfahren nach Anspruch 8, wobei der primäre monoklonale Anti-Protein E6 Antikörper ein monoklonaler Anti-Protein E6 Antikörper der viralen Typen HPV16 und HPV18 der Maus ist, und der primäre monoklonale Anti-Protein E7 Antikörper ein monoklonaler Anti-Protein E7 Antikörper des viralen Typs HPV16 der Maus ist, und der primäre monoklonale Anti-Protein p53 Antikörper ein monoklonaler anti-human p53-Protein Antikörper der Maus ist, und der primäre monoklonale anti-pRb-Protein Antikörper ein monoklonaler anti-human pRb-Protein Antikörper der Maus ist.

12. Verfahren zum Nachweisen einer neoplastischen Zellumwandlung, die durch ein humanes Papillomavirus (HPV) nach einem der Ansprüche 2-5 und 9-12 induziert wird, wobei das Verfahren im Rahmen eines Vorsorgeprogramms einer weiblichen Bevölkerung eingesetzt wird, oder in einem Programm zur Frühdiagnose, für den Nachweis von Gebärmutterhalskrebs, nach einer zytologischen Diagnose von atypischen Plattenzellen (ASC) oder schlimmer, und einem nachfolgenden positiven Ergebnis des hr-HPV-DNA-Tests.

13. Verfahren zum Nachweisen einer neoplastischen Zellumwandlung, die durch ein humanes Papillomavirus (HPV) nach einem der Ansprüche 2-5 und 9-12 induziert wird, wobei das Verfahren im Rahmen eines Vorsorgeprogramms einer weiblichen Bevölkerung eingesetzt wird, oder in einem Programm zur Frühdiagnose, für den Nachweis von Gebärmutterhalskrebs, nach einem positiven Ergebnis des hr-HPV-Tests, und nachfolgender zytologischen Diagnose von atypischen Plattenzellen (ASC) oder schlimmer.

14. Verfahren zum Nachweisen einer neoplastischen Zellumwandlung, die durch ein humanes Papillomavirus (HPV) nach einem der Ansprüche 2-5 und 9-12 induziert wird, wobei das Verfahren im Rahmen von diagnostischer Aktivität, einer Vorsorgeuntersuchung oder einer Nachuntersuchung von Patienten mit neoplastischen HPV-induzierten Läsionen oder solchen Verdachtsfällen, in Gebieten außerhalb der Gebärmutter, wie dem Anus-Genital-Bereich, dem Oropharynx-Bereich, eingesetzt wird.

## Revendications

1. Procédé de détection d'une transformation cellulaire néoplasique induite par le papillomavirus humain (HPV) chez des femmes subissant un dépistage du carcinome du col de l'utérus, comprenant la détection, dans des échantillons de cellules prélevées de la jonction squamo-cylindrique de l'épithélium du col de l'utérus d'une patiente examinée, de la présence d'un complexe protéique E6/p53 fabriqué par la protéine E6 avec la protéine p53 et d'un complexe protéique E7/pRb fabriqué par la protéine E7 avec la protéine pRb, dans lequel la détection desdits deux complexes protéiques met en évidence que chez la patiente examinée la carcinogenèse est devenue irréversible, ladite détection étant réalisée par l'utilisation combinée d'anticorps anti-protéine E6, d'anticorps anti-protéine E7, d'anticorps anti-protéine p53 et d'anticorps anti-protéine pRb.

2. Procédé selon la revendication 1, comprenant les étapes suivantes selon la technique du Western Blot:
a) préparation d'un échantillon de cellules prélevées de la jonction squamo-cylindrique de l'épithélium du col de l'utérus d'une patiente examinée, et son placement dans une solution physiologique ;
b) extraction de protéines dudit échantillon et dénaturation des protéines obtenues ;
c) soumission des protéines dénaturées ainsi obtenues à une électrophorèse sur gel de polyacrylamide ;
d) transfert des protéines obtenues de l'étape précédente sur une membrane et neutralisation des sites libres de ladite membrane ;
e) identification desdites protéines par incubation de celles-ci avec au moins les anticorps suivants :
anticorps monoclonal primaire anti-protéine E6 ;
anticorps monoclonal primaire anti-protéine E7 ;
anticorps monoclonal primaire anti-protéine p53 ;
anticorps monoclonal primaire anti-protéine pRb ;
puis incubation avec des anticorps secondaires anti-IgG conjugués à la biotine ou à une enzyme messager telle que la phosphatase alcaline ;
f) soumission des produits de l'étape précédente à une détection colorimétrique ou à une détection par chimiluminescence ;
dans lequel les résultats de ladite détection présentant une double bande de réaction pour les protéines E6 et E7, ladite double bande comprenant une première bande correspondant au poids moléculaire de la protéine recherchée, en d'autres termes E6 ou E7, et une seconde bande correspondant à un poids moléculaire distinct et supérieur au poids moléculaire de la protéine respective de la première bande, ledit poids moléculaire étant sensiblement égal à 71 kD pour le complexe protéique E6/p53 et à 115,5 kD pour le complexe protéique E7/pRb, indiquent que chez la patiente examinée la carcinogenèse est devenue irréversible ; et/ou les résultats de ladite détection mettant en évidence une double bande de réaction pour au moins l'une des protéines p53 et pRb, ladite double bande comprenant une première bande correspondant au poids moléculaire de la protéine recherchée, p53 ou pRb, et une seconde bande correspondant à un poids moléculaire distinct et supérieur au poids moléculaire de la protéine respective de la première bande, indiquent que chez la patiente examinée, la carcinogenèse est devenue irréversible.

3. Procédé selon la revendication 2, dans lequel les résultats de ladite détection mettant en évidence une bande de réaction unique pour les deux protéines E6 et E7, ladite bande unique correspondant au poids moléculaire de la protéine recherchée E6 ou E7, indiquent que chez la patiente examinée la carcinogenèse n'a pas encore commencé et n'est pas devenue irréversible.

4. Procédé selon la revendication 3, dans lequel les résultats de ladite détection qui ne présentent pas de bande de réaction pour les protéines E6 et E7 indiquent qu'aucune intégration d'ADN viral avec l'ADN cellulaire n'a eu lieu chez la patiente examinée.

5. Procédé selon la revendication 2, dans lequel les anticorps secondaires anti-IgG de ladite étape e) sont conjugués à la phosphatase alcaline, et dans lequel ladite détection de l'opération f) est une détection colorimétrique ou une détection par chimiluminescence.

6. Procédé selon la revendication 2 dans lequel ledit anticorps monoclonal primaire anti-protéine E6 est un anticorps de souris anti-protéine E6 des types viraux HPV16 et HPV18, et ledit anticorps monoclonal primaire anti-protéine E7 est un anticorps de souris anti-protéine E7 des types viraux HPV16 et HPV18.

7. Procédé selon la revendication 2, dans lequel ledit anticorps monoclonal primaire anti-protéine p53 est un anticorps monoclonal de souris anti-protéine p53 humaine, et ledit anticorps monoclonal primaire anti-protéine pRb est un anticorps monoclonal de souris anti-protéine pRb humaine.

8. Procédé selon la revendication 1, dans lequel ladite détection est réalisée par l'utilisation combinée d'anticorps anti-protéine E6, d'anticorps anti-protéine E7, d'anticorps anti-protéine p53 et d'anticorps anti-protéine pRB, comprenant les étapes suivantes conformément à la technique ELISA en Sandwich :
a) identification d'un échantillon à examiner, stocké conformément à la cytologie en phase liquide (LBC) ;
b) adsorption, dans différents puits d'une plaque de microtitration, d'anticorps anti-protéine E6 et d'anticorps anti-protéine E7, en tant qu'anticorps de capture de la protéine virale E6 ou E7 ;
c) blocage des sites restants du puits en liant les protéines par un tampon de blocage ;
d) distribution directement dans chaque puits de l'échantillon de cellules indiqué à analyser ;
e) identification des protéines d'intérêt par incubation avec un anticorps anti-protéine p53 dilué dans les puits dans lesquels un anticorps anti-protéine E6 est adsorbé et avec un anticorps anti-protéine pRb dilué dans les puits dans lesquels un anticorps anti-protéine E7 est adsorbé ;
f) blocage du substrat endogène ;
g) incubation dans les puits respectifs de l'anticorps conjugué à la peroxydase de raifort (HRP) ;
h) distribution d'une solution chromogène dans chaque puits :
i) soumission du produit de réaction de l'étape h) à une lecture de l'intensité lumineuse détectée à une longueur d'onde de 450 nm, par un spectrophotomètre,
dans lequel des résultats mettant en évidence la coloration survenue, documentée par une absorbance positive, indique que chez la patiente examinée les complexes protéiques E6/p53 et E7/pRb sont présents, confirmant l'interaction entre des protéines virales et humaines, et indiquant par conséquent que chez la patiente examinée la carcinogenèse a commencé et est devenue irréversible.

9. Procédé selon la revendication 8, dans lequel dans les étapes e), f), g) et h), les anticorps anti-protéine P53 et pRB biotinylés sont utilisés et détectés avec de la streptavidine conjuguée à la peroxydase ou une autre enzyme pour amplifier le signal chromogène.

10. Procédé selon la revendication 8, dans lequel dans le cas où les résultats de détection ne montrent aucune positivité de réaction, avec une absence d'absorbance de signal à 450 nm, pour les deux complexes protéiques E6/p53 et E7/pRb, le résultat indique qu'aucune intégration de l'ADN viral avec l'ADN cellulaire ne s'est produite chez la patiente examinée ; sans exclure la possibilité de se trouver en présence d'une intégration de l'ADN viral avec l'ADN cellulaire.

11. Procédé selon la revendication 8, dans lequel ledit anticorps monoclonal primaire anti-protéine E6 est un anticorps monoclonal de souris anti-protéine E6 des types viraux HPV16 et HPV18, ledit anticorps monoclonal primaire anti-protéine E7 est un anticorps monoclonal de souris anti-protéine E7 du type viral HPV16, ledit anticorps monoclonal primaire anti-protéine p53 est un anticorps monoclonal de souris anti-protéine p53 humaine, ledit anticorps monoclonal primaire anti-protéine pRb est un anticorps monoclonal de souris anti-protéine pRb humaine.

12. Procédé de détection d'une transformation cellulaire néoplasique induite par le papillomavirus (HPV) humain selon l'une quelconque des revendications 2 à 5 et 9 à 12, dans lequel ledit procédé est inséré dans le cadre d'un programme de dépistage d'une population féminine, ou dans un programme de diagnostic précoce, pour la détection d'un carcinome du col de l'utérus, après un diagnostic cytologique de cellules squameuses atypiques (ASC) ou pire, et un résultat positif subséquent du test ADN HPV-hr.

13. Procédé de détection d'une transformation cellulaire néoplasique induite par le papillomavirus (HPV) humain selon l'une quelconque des revendications 2 à 5 et 9 à 12, dans lequel ledit procédé est inséré dans le cadre d'un programme de dépistage d'une population féminine, ou dans un programme de diagnostic précoce, pour la détection d'un carcinome du col de l'utérus, après un résultat positif du test ADN HPV-hr, et un diagnostic cytologique subséquent de cellules squameuses atypiques (ASC) ou pire.

14. Procédé de détection d'une transformation cellulaire néoplasique induite par le papillomavirus (HPV) humain selon l'une quelconque des revendications 2 à 5 et 9 à 12, dans lequel ledit procédé est inséré dans le cadre d'une activité de diagnostic, du dépistage ou du suivi de patientes présentant, ou chez qui l'on suspecte, des lésions néoplasiques induites par le HPV, dans des zones extra-utérines, par exemple la région ano-génitale, la région oropharyngée.
